# EUROPEAN PATENT APPLICATION

(11) **EP 2 298 277 A1**
(43) Date of publication of application: **23.03.2011**
(21) Application number: 09011533.8
(22) Date of filing: 09.09.2009
(51) Int. Cl.: A61K 9/00, A61K 9/70

(54) **Transdermal patch formulation**

(71) Applicant: Labtec GmbH, 40764 Langenfeld (DE)
(72) Inventor: Braun, Sebastian, Dr., D-42929 Wermelskirchen (DE); Breitenbach, Armin, Dr., D-51371 Leverkusen (DE); Becker, Ulrich, Dr., D-40764 Langenfeld (DE)
(74) Representative: Von Renesse, Dorothea

(57) **Abstract**

A method for preparing a transdermal patch comprising a substrate layer is provided, the method comprising the steps of contacting an active pharmaceutical ingredient with a retaining means to provide a composition, applying the composition obtained to a carrier material to form a substrate layer of the transdermal patch, wherein the retaining means remain within the final transdermal patch.

## Description

The invention relates to the field of pharmaceutical patch formations, in particular patch formulations containing rasagiline or derivatives or analogues thereof.

Rasagiline, a selective inhibitor of monoamine oxidase-B, is known for the treatment of central nervous system diseases such as Parkinson's disease (PD), depression, etc., and has gone on sale in Europe. The chemical structure of rasagiline is as follows:

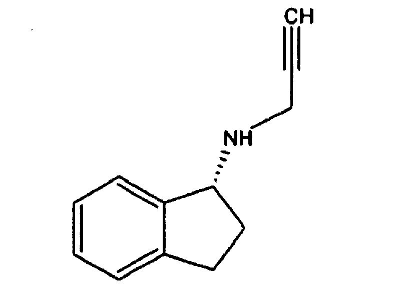

Rasagiline is the active ingredient in the anti Parkinson medicament market in Europe under the brand name Azilect®. This is a tablet which contains a salt of rasagiline, namely the rasagiline mesylate. The tablet has to be taken once daily.

Since the majority of Parkinson patients have difficulties in walking and oral taking, it is desirable to formulate rasagiline in a transdermal administration so that rasagiline may be administered less frequently than every other day or more. Desirable is an administration which is for example only twice or once a week.

The administration of an active ingredient as a patch formulation requires that the active ingredient can sufficiently penetrate the skin. In case of rasagiline this is thought to require the use of the free base, since the solubility of the salt thereof in a lipophilic polymer matrix used in transdermal patch formulation as well as its penetration into the skin is too little.

WO2007/101400 A1 describes a transdermal patch formulation containing rasagiline salt. In order to overcome the drawbacks thereof in terms of limited solubility and penetration rate into the skin, this patent application suggests to further add a penetration regulator and a penetration enhancer into the patch formulation. If rasagiline free base is contained in the formulation of WO2007/101440 A1 it is *in situ* converted from the rasagiline salt into the free base by the addition of bases. However this conversion is disadvantageous since it requires additional steps in the manufacture process and furthermore raises unwanted regulatory hurdles since the conversion may be incomplete.

The patch formulation of W02007/101400 A1 can either be manufactured according to the standard hot melt technique or according to the standard solvent/casting/drying process. In the hot melt technique, the active ingredient is added to a melted polymer material at a temperature of 50 to 200°C. The molten material is then applied to an inert backing layer and cooled to form the substrate layer. In the solvent/casting/drying technique, the active ingredient is dispersed in a volatile solvent first, and then an organic polymer material is added to the solution. This mixture then is used to form the substrate layer, whereby the solvent is evaporated.

The inventors now found, that applying these techniques to the manufacture of a patch formulation containing the free base of rasagiline inevitably leads to a substantial loss of the active ingredient, which can even be up to 90 or 95% of the originally introduced amount of the active ingredient. This is most likely due to the volatility of the active ingredient causing the active ingredient to sublime or to evaporate during the manufacturing process.

Thus, it is objective of the present invention to provide novel means for the manufacture of transdermal patch formulations, in particular patch formulations containing an active ingredient/drug substance with a high volatility, in particular the free base of rasagiline, which allows for a reduced loss of active ingredient. In particular this manufacture process should be simple and easy to perform.

This objective is solved by the method according to claim 1. Further embodiments of the invention are subject matter of additional independent or dependent claims. Furthermore novel and favourable patch formulations are suggested.

The core concept of the invention is based on the findings that an incubation of the volatile active ingredient in retaining means before admixing the active ingredient into the polymer matrix ("carrier material") of the patch formulation results in a substantially reduced loss of active ingredient during the manufacture process. Due to this preincubation of the active ingredient in a retaining means the recovery rate of the active ingredient in the final patch formulation is significantly increased compared to the known processes. The recovery rate according to the invention is at least 20%, favourably at least 50% or 70 %. Most preferably the recovery rate is at least 80% or even 100%.

Advantageously the transdermal patch according to the invention containing the retaining means does not require any further additive such as penetration enhancers or penetration regulators to provide a sufficient penetration of the active ingredient into the skin.

The method according to the invention is suitable for any active ingredients or mixtures thereof. However, it is preferably used to prepare transdermal patches with volatile active pharmaceutical ingredients, in particular carbamates such as rasagiline or its derivatives or analogues, preferably in the form of the free base. A volatile active pharmaceutical ingredient is any drug substance either with a melting point at room temperature of below 100°C and/or which evaporates at a temperature of more than 30°C, more than 50°C or more than 70°C.

The active pharmaceutical ingredient used in the present invention preferably has a chemical structure according to one of the following formulas:
Formula I: 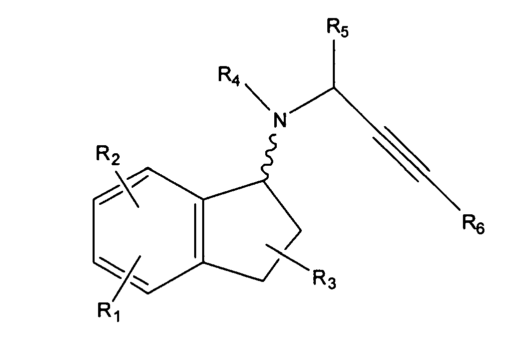 wherein
   R₁ is hydrogen, halogen, alkyl, alkoxy, acyl, acyloxy, aryl, aralkyl, hydroxy, carboxy, amine, alkylamine, dialkylamine, nitro, or -OC(O)NR₁₂R₁₃, and may be substituted by one or more substituents selected from alkyl, halogen, hydroxy, carboxy, amine, alkylamine, dialkylamine;
   R₂, R₃, R₄, R₅, R₆, R₁₂ and R₁₃ independent from each other are hydrogen, halogen or alkyl.

One or more, preferably one or two, of the carbon atoms in the formula including the substituents may be replaced by a heteroatom such as nitrogen, oxygen or sulfur. Preferably, R₁ is hydrogen or -OC(O)NR₁₂R₁₃, wherein R₁₂ and R₁₃ preferably are methyl and/or ethyl, more preferably R₁₂ is methyl and R₁₃ is ethyl. R₂, R₃, R₄, R₅ and R₆ each preferably are hydrogen.
Formula II: 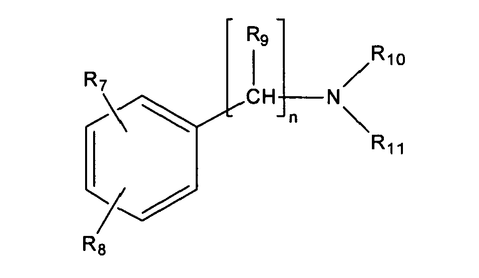 wherein
   R₇ is hydrogen, halogen, alkyl, alkoxy, acyl, acyloxy, aryl, aralkyl, hydroxy, carboxy, amine, alkylamine, dialkylamine, nitro, or -OC(O)NR₁₄R₁₅, and may be substituted by one or more substituents selected from alkyl, halogen, hydroxy, carboxy, amine, alkylamine, dialkylamine;
   R₈, R₉, R₁₀, R₁₄ and R₁₅ independent from each other are hydrogen, halogen or alkyl, wherein if more than one R₉ is present, these R₉ groups may also be different from each other;
   R₁₁ is hydrogen, halogen or alkyl optionally substituted by halogen or hydroxy;
   n is an integer from 1 to 4, preferably 1 or 2.

One or more, preferably one or two, of the carbon atoms in the formula including the substituents may be replaced by a heteroatom such as nitrogen, oxygen or sulfur. Preferably, R₇ is hydrogen or -OC(O)NR₁₄R₁₅, wherein R₁₄ and R₁₅ preferably are methyl and/or ethyl, more preferably R₁₄ is methyl and R₁₅ is ethyl. R₈ preferably is hydrogen. R₉ preferably is hydrogen or methyl. n preferably is 1 or 2. The group -[CHR₉]ₙ- preferably is -CH₂-CH(CH₃)- or -CH(CH₃)-. R₁₀ preferably is 2-propynyl (-CH₂-C≡CH) or methyl. R₁₁ preferably is methyl.

Razemic mixtures as well as isolated stereochemical forms of the above structures are encompassed by the formulas, including any isomers, stereoisomers, diastereoisomers and enantiomers.

Halogen preferably refers to fluorine, chlorine, bromine or iodine. Alkyl refers to a saturated or partially unsaturated, linear or branched carbohydrate chain preferably comprising 1 to 20 carbon atoms, more preferably 1 to 10, or 1 to 6 carbon atoms, and most preferably 1 to 4 carbon atoms. Alkyl includes unsaturated carbohydrate chains, i.e. alkenyl and alkynyl, having one or more carbon-carbon double bonds and/or one or more carbon-carbon triple bonds. Examples of alkyl are methyl, ethyl, n-propyl, isopropyl, n-butyl, tert. butyl, pentyl, hexyl, vinyl, and 2-propynyl. Alkoxy refers to the group -O-alkyl. Acyl refers to -C(O)-alkyl. Acyloxy refers to the group -OC(O)-alkyl. Aryl refers to an aromatic carbocyclic group preferably having 6 to 20 carbon atoms, more preferably 6 to 10 carbon atoms, having a single ring or multiple rings or multiple condensed rings. Examples are phenyl, naphthyl and biphenyl. Aralkyl refers to the group -alkyl-aryl.

The active pharmaceutical ingredient preferably is rasagiline or a derivative or analogue thereof. Exemplary derivatives or analogues of rasagiline are:
Ladostigil (TV3326, [(N-propargyl)-(3R)-aminoindan-5-yl]-ethyl methyl carbamate) 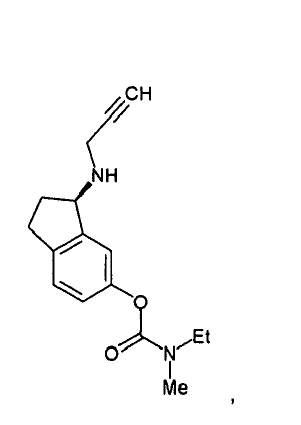 TV3279 [(N-propargyl)-(3S)-aminoindan-5-yl]-ethyl methyl carbamate

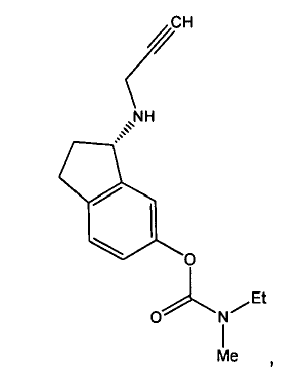

Selegiline (R)-N-methyl-N-(1-phenylpropan-2-yl)prop-2-yn-1-amine

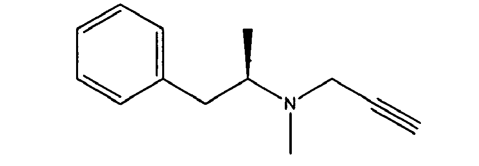

Rivastigmine

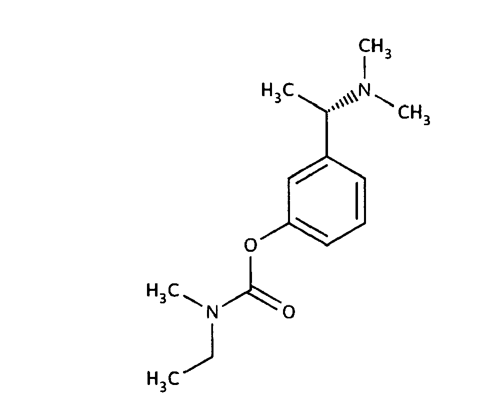

The retaining means are defined as any substance matter which either chemically or physically interacts with the active ingredient and therewith prevents its evaporation or sublimation from the polymer matrix, in particular during the manufacturing process. "Preventing evaporation or sublimation" in the context of the invention means, that the recovery rate of the active ingredient at room temperature and after the manufacture process is at least 20 %, favourably at least 50% or 70 %. Most preferably the recovery rate is at least 80% or even 100%.

The retaining means may be a solid or liquid substance having e.g. the active pharmaceutical ingredient attached or bound to its surface or incorporated into its body.

Suitable liquid retaining means are e.g. selected from the group consisting of natural or synthetic oils, fatty alcohols such as e.g. polidocanol, isopropyl myristate, polyalkoxylated fatty acids and polyalkoxylated fatty alcohols (in particular polyethoxylated fatty acids and fatty alcohols) with lower molecular weight, i.e. a molecular weight which renders them liquid at room temperature, and mixtures thereof.

Suitable solid retaining means are selected e.g. from the group consisting of PVP, PEO, PVA, cellulose or derivatives, starch or derivatives, PVPVA or their blends, polyalkoxylated fatty acids and polyalkoxylated fatty alcohols (in particular polyethoxylated fatty acids and fatty alcohols) with higher molecular weight, i.e. a molecular weight which renders them solid at room temperature, and mixtures thereof. Solid and liquid retaining means can also be mixed. The physical state of the retaining means (liquid/solid) is well known to a person skilled in the art being a function of molecular weight.

The retaining means may be covered or coated with the active pharmaceutical ingredient in any manner known in the art, for example by applying the active pharmaceutical ingredient in a molten state and solidifying it, by applying a solution of the active pharmaceutical ingredient and drying it, or by any other method. The incorporation of the active pharmaceutical ingredient into the retaining means may be achieved by melting both materials, mixing them and cooling the mixture, or by solving both materials in the same solution and drying (e.g. spray-drying) the solution. The resulting composition can then be applied to the polymer matrix.

In a preferred embodiment the retaining means is a liquid with a low volatility ("low volatile solvent"). This is defined as any substance that is liquid from room temperature up to 70°C.

As a carrier material ("polymer matrix") any common or suitable material may be used. The mixture including the active ingredient and the retaining means are added to the polymer matrix to form the "substrate layer" of the final transdermal patch.

Preferably, the carrier material comprises at least one type of the following polymer materials: polyacrylates and derivatives thereof, silicone polymers and derivatives thereof, polyisobutylene and derivatives thereof, ethylene-vinyl acetate copolymers and derivatives thereof, Styrene-block-co-polymers and derivatives thereof, polyacrylic acids and derivatives thereof, polyoxazolines (POX) and derivatives thereof, poylurethanes and derivatives thereof, polyolefines and derivatives thereof, polyesters and derivatives thereof.

The carrier material may be adhesive, thus constituting the support layer for the active ingredient as well as well as the adhesive layer for the adherence of the patch to the skin. It can also be non-adhesive. Then a separate adhesive layer has to be included into incorporated into the transdermal patch.

In one embodiment of the invention, the retaining means may be a liquid. In this case, the active pharmaceutical ingredient can be solved or dispersed first in the retaining means, forming a solution or suspension. Then the carrier matrix is soaked with the solution or suspension. To aid the soaking process, well known soaking additives such as Plastoid B, Eudragit polymers, SiO₂, PEO, PVP, PVA, cellulose or derivatives, starch and derivatives, cyclodextrins and the like, either alone or in combination may be added to the carrier material. Furthermore, the solution or suspension may be made viscous by adding an additive such as a non-adhesive carrier material and may then be applied to the surface of the carrier matrix.

In preferred embodiments, the solid composition is ground. The ground material may then be dispersed in a melt or solution of the carrier material, followed by the cooling or drying of the mixture to form the substrate layer of the transdermal patch. Alternatively, the solid composition comprising the retaining means and the active pharmaceutical ingredient may be applied to the solid carrier material being adhesive. Here, the composition may either be applied as granulate material or powder (e.g. after grinding) or as bulk material (e.g. as fleece material).

The transdermal patch prepared by the methods according to the invention may further comprise a backing layer, preferably an inert backing layer, i.e. a backing layer which does not interfere with the activity or bioavailability of the active pharmaceutical ingredient, a protective layer, i.e., e.g., a layer protecting the bottom of the transdermal patch during storage which has to be peeled off prior to use, and/or a release controlling layer which regulates the rate of release of the active ingredient. The backing layer (overlay) can e.g. be a foil or a film of polyethylene or polypropylene or a non-woven fabric. The protective layer (antistick layer, release liner) can also be a foil, or a complex film formed by materials such as polyethylene or polypropylene or polycarbonate, etc, or a thick slick paper pretreated with paraffin oil or siliconized or fluoro coated. The release controlling layer may be a membrane having a defined pore size.

In one embodiment of the invention, the retaining means is a low volatile liquid plasticizer and the carrier material is a mixture of an adhesive polymer and a non-adhesive polymer. The carrier material preferably is chosen such that it exhibits a rather low adhesiveness. Upon soaking of the carrier material with the retaining means comprising the active pharmaceutical ingredient, the carrier material is softened by the plasticizer and its adhesiveness is increased to a suitable level.

In preferred embodiments, no penetration enhancer in addition to the retaining means - which itself may be a penetration enhancer - is added to the transdermal patch. More preferably no penetration enhancer at all is added to the transdermal patch.

In the following, particular embodiments of the method according to the invention are described:
One embodiment comprises the steps of
   a) adsorbing the active pharmaceutical ingredient onto the solid retaining means to form a composition, preferably by melting the active pharmaceutical ingredient in presence of the retaining means and then solidifying;
   b) grinding the composition;
   c) mixing the ground composition with molten carrier material or with a solution comprising the carrier material;
   d) applying the dispersion or solution onto a backing layer to form a film; and
   e) cooling or drying the film to form a substrate layer.

In a further embodiment the method comprises the steps of:
a) forming an adhesive matrix layer comprising the carrier material;
b) mixing the active pharmaceutical ingredient with the liquid retaining means to form a solution or suspension;
c) applying the solution or suspension to the matrix layer to form a substrate layer, preferably by soaking, printing or casting.

In yet another embodiment the method comprises the steps of:
a) forming an adhesive matrix layer comprising the carrier material;
b) mixing the active pharmaceutical ingredient with the liquid retaining means and a non-adhesive carrier material to form a viscous solution or suspension;
c) applying the solution or suspension to the matrix layer to form a substrate layer, preferably by spreading; and
d) optionally applying another adhesive layer.

In a further embodiment the method comprises the steps of:
a) forming an adhesive matrix layer comprising the carrier material;
b) attaching the solid retaining means, preferably a fleece material, to the adhesive matrix layer;
c) applying the active pharmaceutical ingredient to the retaining means, for example by impregnating, either before or after the retaining means is attached to the adhesive matrix layer; and
d) applying another adhesive layer to the other site of the retaining means.

In another embodiment the method comprises the steps of:
a) forming an adhesive matrix layer comprising the carrier material;
b) adsorbing the active pharmaceutical ingredient onto the solid retaining means to form a composition, preferably by melting the active pharmaceutical ingredient in presence of the retaining means and then solidifying;
c) grinding the composition;
d) applying the ground composition to the adhesive matrix layer to form a substrate layer; and
e) optionally applying another adhesive layer.

Furthermore, the present invention relates to a method for preparing a transdermal patch comprising a substrate layer, the method comprising the steps of:
a) melting a carrier material;
b) quickly dispersing an active pharmaceutical ingredient into the molten carrier material;
c) forming a substrate layer with the mixture obtained in step (a); and
d) quickly cooling the substrate layer to solidify it.

In this method, the carrier material preferably is an adhesive, the substrate layer preferably is formed on a backing layer and/or the substrate layer preferably is cooled by forming it on a cooled surface. Furthermore, each of the specific embodiments described herein with respect to the methods for preparing a transdermal patch also apply to this method, if appropriate.

In another aspect, the present invention provides a transdermal patch comprising a backing layer and a substrate layer comprising a volatile active pharmaceutical ingredient, a carrier material and a retaining agent. Preferably, the transdermal patch is obtainable or prepared by any one of the methods according to the invention. Furthermore, the embodiments described herein with respect to the preparation methods also apply accordingly to the transdermal patch according to the invention.

In preferred embodiments, the transdermal patch does not contain a penetration enhancer in addition to the retaining means which itself may be a penetration enhancer. More preferably, the transdermal patch does not contain any penetration enhancer at all.

The transdermal patch preferably contains at least 1% (w/w) of the retaining means. Furthermore, it preferably contains at least 0.1% (w/w) of the active pharmaceutical ingredient.

In one embodiment, the transdermal patch according to the invention is for use in medicine. In particular, it contains rasagiline or a derivative thereof as the active pharmaceutical ingredient and is for treatment or prophylaxis of a nervous system disease, preferably a nervous system disease selected from the group consisting of Parkinson's disease, Alzheimer's disease, depression, hyperactive child syndrome, restless leg syndrome, multiple sclerosis and abstinence syndrome.

Thus, in one specific embodiment the invention provides a rasagiline transdermal patch for treatment or prophylaxis of nervous system diseases, wherein the patch comprises an inert backing layer chemically inert to substrate ingredients, a substrate layer comprising the free base of rasagiline and a protective layer to be peeled off before use, wherein the substrate layer is a drug-carrying reservoir comprising an organic polymer that is soaked with pharmaceutically acceptable low volatile solvent containing rasagiline or that is dispersed with a ground absorber material comprising rasagiline.

The inventors have found that that a transdermal patch (TDS) comprising an organic matrix which is soaked with a low volatile solvent solubilising the rasagiline permits a highly efficient skin penetration. Furthermore, rasagiline in TDS formulations according to this invention exhibits the stability that is required for a pharmaceutical formulation.

This skin penetration was tested in an in vitro method using excised human skin, representing a model that is highly relevant for the human clinical situation.

Due to the use of a low volatile solvent a subsequent drying step is not necessary. This results in a more efficient production process and prevents the rasagiline and the patch material from drying-associated damage especially as result of the high drying temperature.

The low volatile solvent can also act as an enhancer for the transdermal absorption of rasagiline. If appropriate, the substrate layer may further comprise a controlled release substrate layer and/or an adhesive layer. If the drug reservoir substrate layer or the controlled release substrate layer has an appropriate adhesiveness, the adhesive layer as a separate layer may be unnecessary

Furthermore, a rasagiline transdermal patch is provided wherein the substrate layer comprises a ground layer containing an organic polymer coated with a high viscosity layer comprising a pharmaceutically acceptable low volatile solvent containing rasagiline.

In the above rasagiline transdermal patch, rasagiline has an effective amount of 0.01 mg/cm² to 50 mg/cm² in the TDS,

The rasagiline transdermal patch of the present invention has an administration area of about 1 cm² to 50 cm² during a transdermal therapy.

The following figures and examples serve the purpose to illustrate the present invention without in any way limiting this scope thereof. However, they relate to preferred embodiments of the present invention.

### BRIEF DESCRIPTION OF THE DRAWINGS

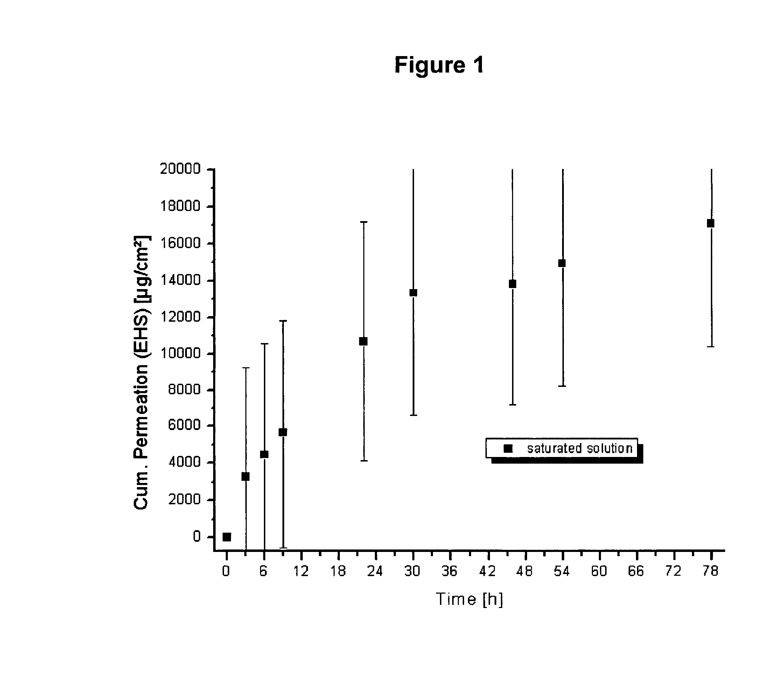

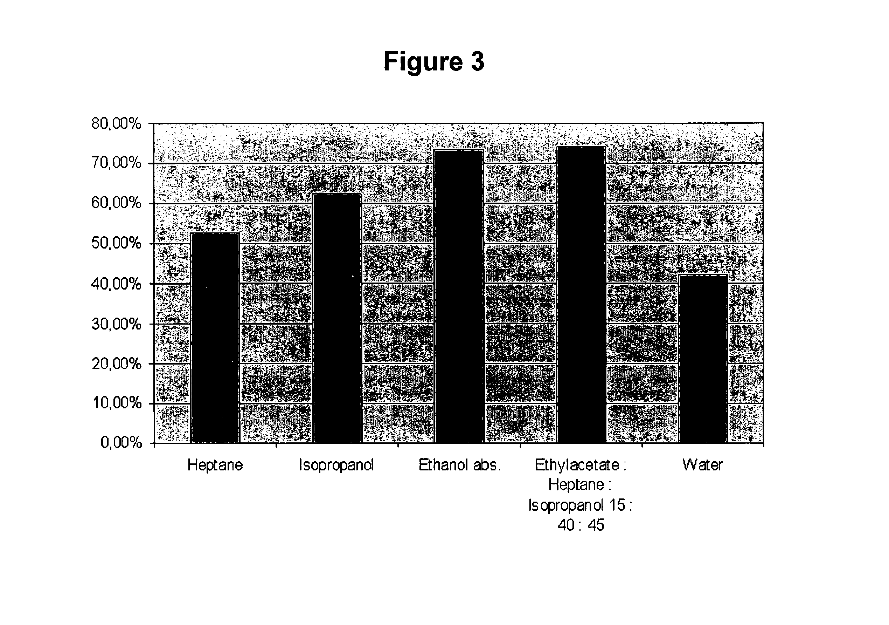

**Figure 1** shows the time-cumulative penetration of a saturated solution of the free base of rasagiline across excised human skin (EHS). A saturated solution of rasagiline base in phosphate buffer pH 6,5 (No 4010800, Ph. Eur. 6.0) was prepared and ist permeation across excised human skin was tested

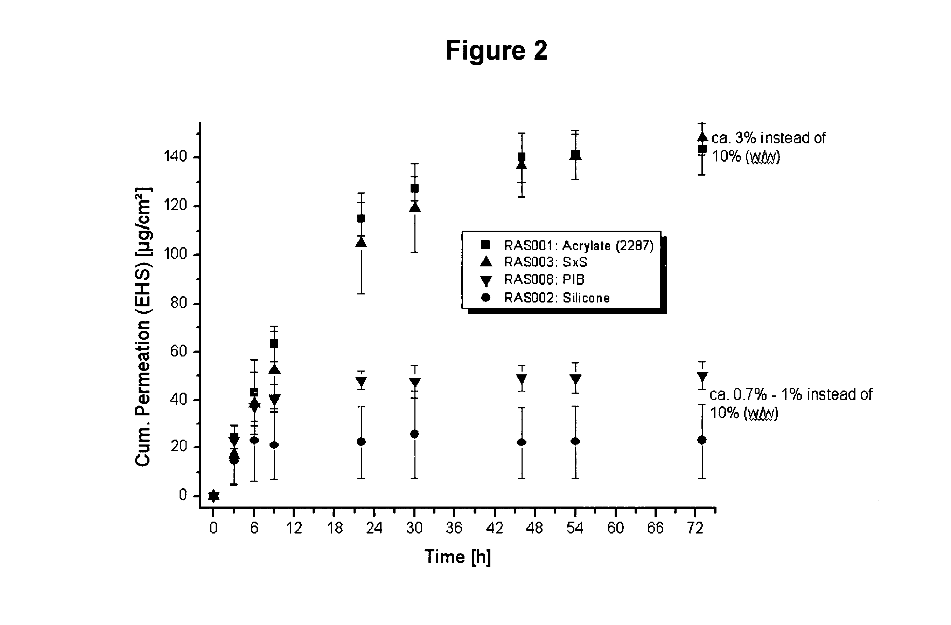

**Figure 2** shows the loss of rasagiline substance during state of the art patch manufacturing methods as revealed by low skin permeation rates (EHS).
   **Acrylate:** Rasagiline base was dissolved in ethyl acetate and added to a solution of durotak 2287 (acrylic adhesive), the mixture was cast to a liner, dried and laminated with a protective foilt to form a standard solvent casting acrylate TDS
   **Silicone:** Two Silicone adhesive solutions (BioPSA 7-4301/7-4201) were mixed. An ethanolic PVP solution was mixed with rasagiline base. This mixture was added to the silicone adhesived solution under stirring, cast onto a liner, dried, covered with a protective liner to form a standard solvent casting silicone TDS
   **SxS (heptane):** Rasagiline base was dissolved in heptane and added to a solution of DuroTak 611a under stirring, cast onto a liner, dried, covered with a protective liner to form a standard solvent casting SxS TDS.
   **PIB:** Rasagiline base was dissolved in ethanolic PVP solution and added to a mixture of high, medium and low MW PIB in hexan under stirring, cast onto a liner, dried, covered with a protective liner to form a standard solvent casting PIB TDS
**Figure 3** shows the loss of rasagiline substance in % of starting material after removal of different solvents by drying. Rasagiline base (50 mg) was dissolved in the presented solvents (0.14 g) and the placed into an over at 70°C to monitor ist loss. Surprisingly, even pure water, which is not a preferred solvent of a lipophilic base cause significant sublimation (=loss) of rasagiline.

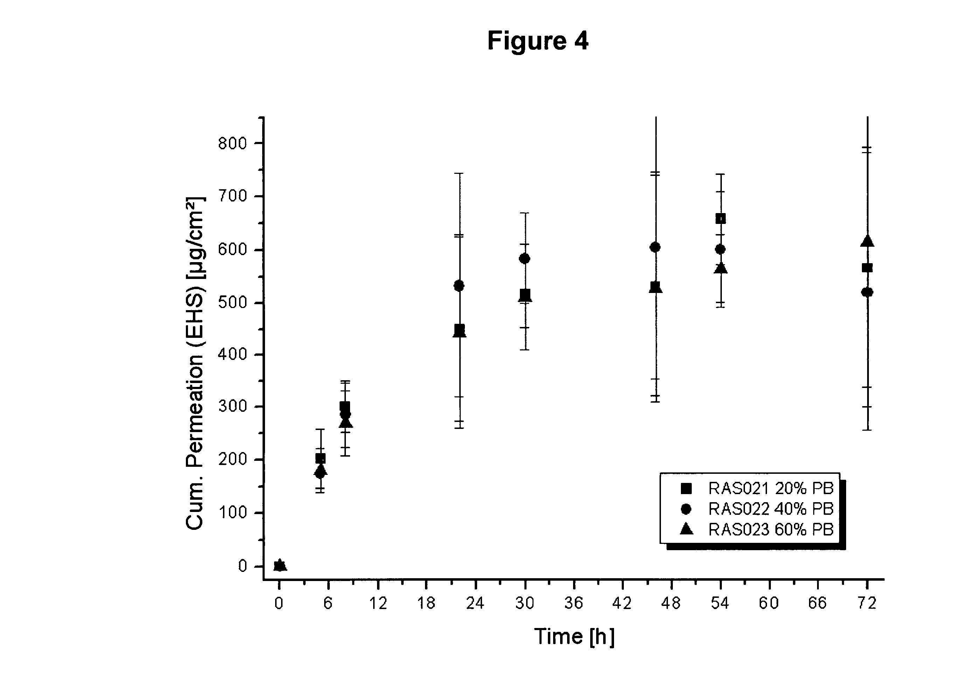

**Figure 4** shows the time-cumulative EHS penetration of a transdermal patch with an acrylate matrix containing 20%(■), 40%,(●) or 60% (A) of Plastoid B and rasagiline solubilised in the low volatile solvent isopropyl myristate. An acrylic adhesive matrix comprising Durotak 2287 further containing either 20, 40 or 60% (w/w) of Plastoid B as absorber were prepared by solvent casting. Rasagiline base solutions were prepared and applied to the above described matrix, after complete soaking of this solution into the matrix, a protective liner was added.A solution of Rasagiline was prepared by dissolving Rasagiline base at room temperature with IPM as solvent. Samples were then tested for rasagiline base permeation from these patches across excised human skin.

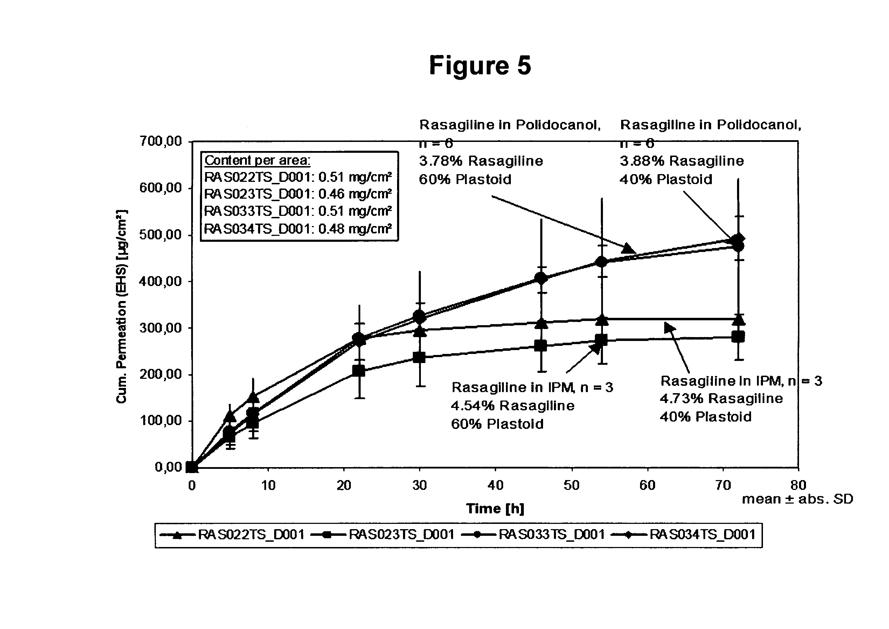

**Figure 5** shows the time-cumulative EHS penetration of a transdermal patch containing:
   (i) 3.78% rasagiline in polidocanol and 60% of Plastoid B (◆);
   (ii) 3.88% rasagiline in polidocanol and 40% of Plastoid B (●);
   (iii) 4.54% rasagiline in isopropyl myristate and 60% of Plastoid B (A);
   (iv) 4.73% rasagiline in isopropyl myristate and 40% of Plastoid B (■).
   An acrylic adhesive matrix comprising Durotak 2287 further containing either 40 or 60% (w/w) of Plastoid B as absorber were prepared by solvent casting. Rasagiline base solutions were prepared and applied to the above described matrix, after complete soaking of this solution into the matrix, a protective liner was added. A solution of Rasagiline was prepared by dissolving Rasagiline base at room temperature with polidocanol as solvent versus IPM as solvent. Samples were then tested for rasagiline base permeation from these patches across excised human skin.

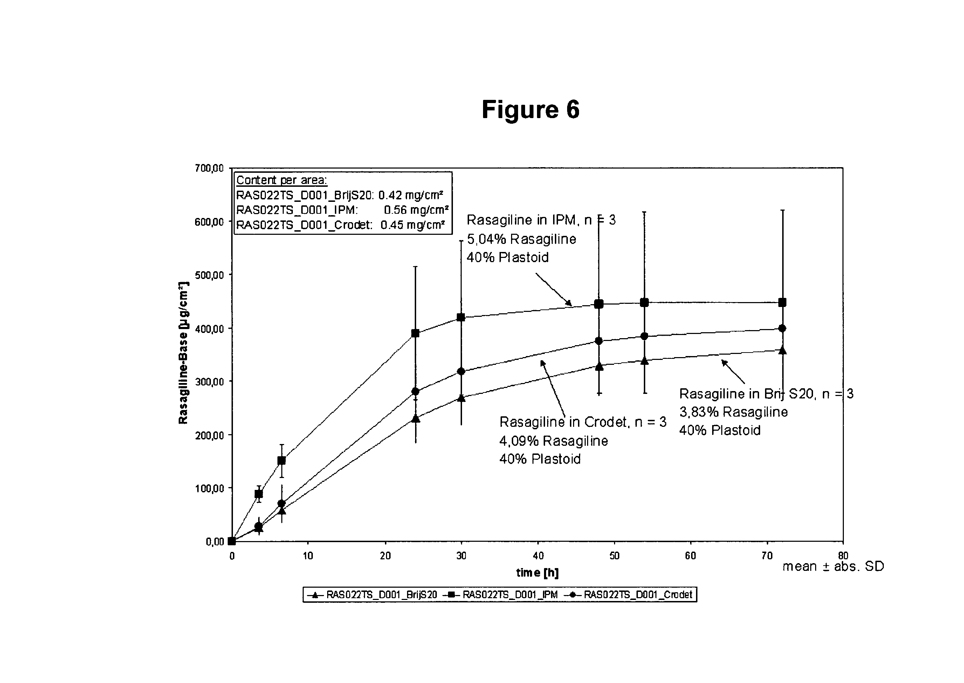

**Figure 6** shows the time-cumulative EHS penetration of a transdermal patch produced by a "printing process" and containing:
   (i) 5.04% rasagiline in isopropyl myristate and 40% of Plastoid B (■);
   (ii) 4.09% rasagiline in Crodet and 40% of Plastoid B (●)
   (iii) 3.83% rasagiline in Brij S20 and 40% of Plastoid B (A)
   An acrylic adhesive matrix comprising Durotak 2287 further containing either 40% (w/w) of Plastoid B as absorber were prepared by solvent casting. Rasagiline base solutions were prepared and applied to the above described matrix, after complete soaking of this solution into the matrix, a protective liner was added. A solution of Rasagiline was prepared by dissolving Rasagiline base at room temperature with IPM as solvent versus Brij or Crodet at slightly elevated temperature as solvent. Samples were then tested for rasagiline base permeation from these patches across excised human skin.

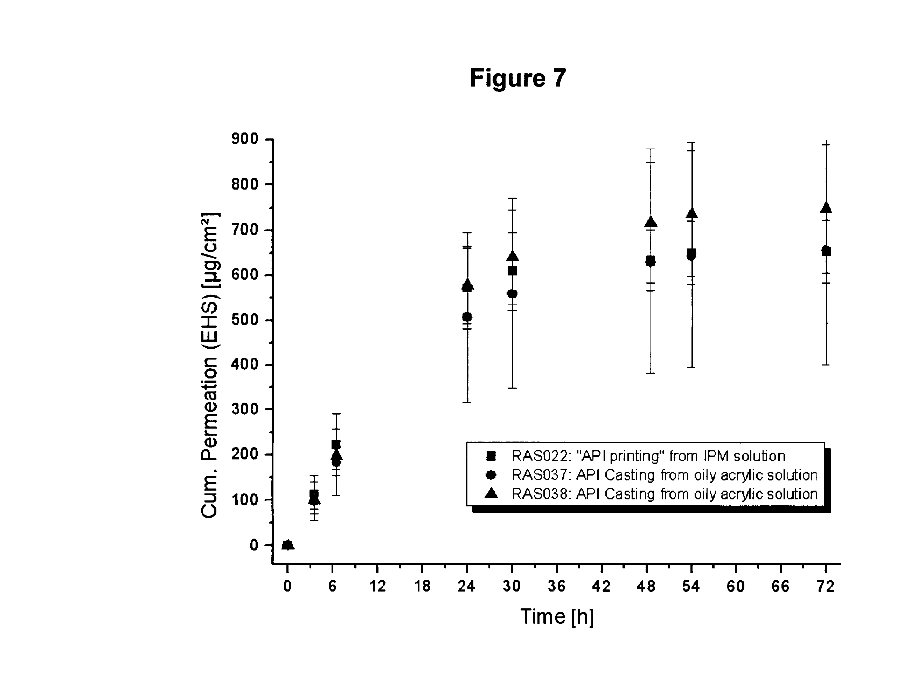

**Figure 7** shows the time-cumulative EHS penetration of a transdermal patch produced by a printing process using an isopropyl myristate solution (■) or by a casting process using an oily acrylic solution (●; ▲).

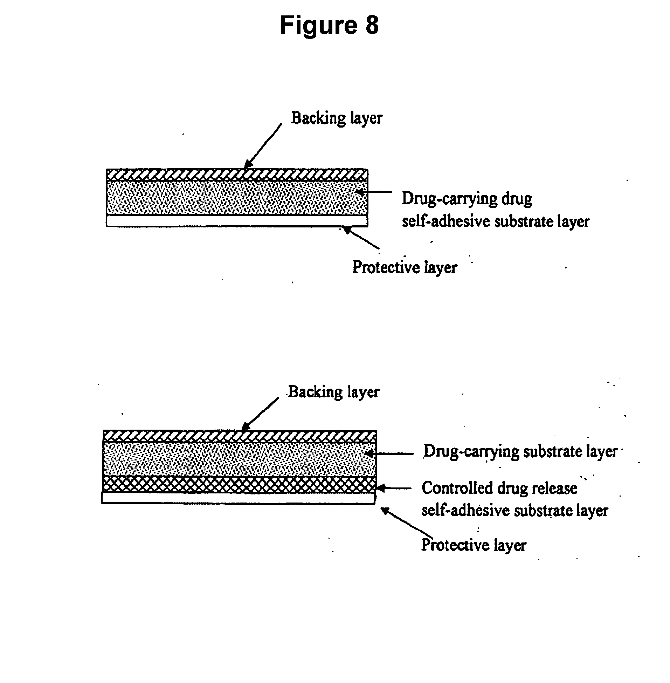

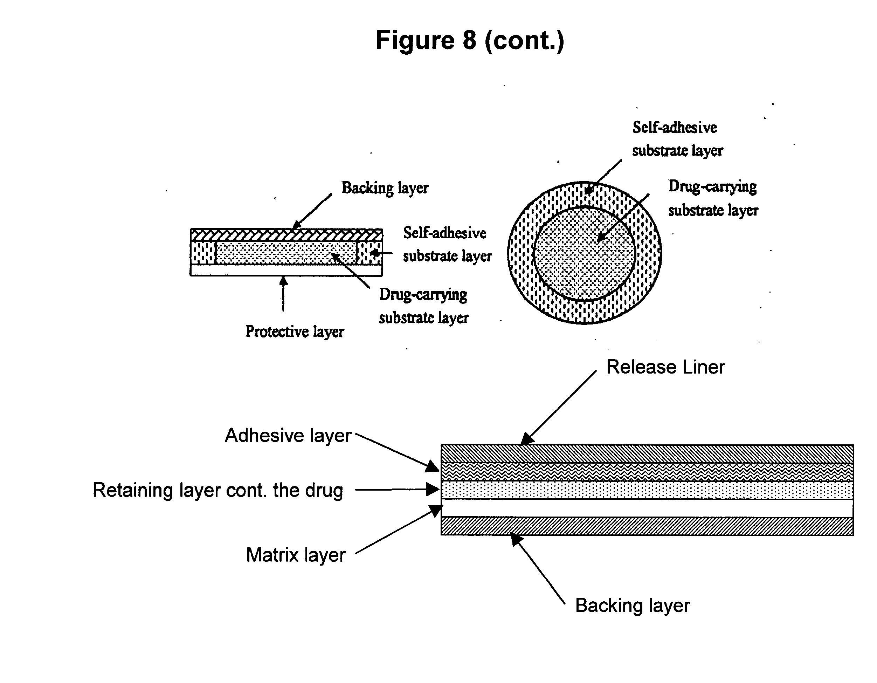

**Figure 8** shows exemplary cross section views of the patches of Examples 1-6.

### EXAMPLES

### EXAMPLE 1: Pilot test regarding the solubility of Rasagiline base in non-volatile solvents for use in a TDS

### Methods and Results

### 1.1 Solubility of Rasagiline base

| Weighted sample ca. 20-25mg | 0,5 g or mL, respectively *Equates to a ca. 1:20 mixture* | 1,0 g or mL, respectively *Equates to a ca. 1:40 mixture* |
|---|---|---|
| Isopropyl myristate (IPM) | Solubilised | Solubilised |
| Cremophor EL | Nearly completely solubilised | Solubilised |
| Vitamin-E-acetate | Nearly completely solubilised | Solubilised |
| Propylenglycol | Solubilised | Solubilised |
| Silicone oil 1000 | Not Solubilised | Solubilised |
| PEG 400 | Solubilised | Solubilised |
| Glycerol anhydrous | Not Solubilised | Not completely solubilised, residual crystals observable |
| paraffine, viscous | Not Solubilised | Solubilised |
| Polysorbat 80 | Solubilised | Solubilised |

### 1.2 Use of isopropyl myristate (IPM) and different adhesives

- The adhesive is streaked and dried, a TDS 4,5cm² is punched out
- ca. 200µl of a IPM solution containing rasagiline (Eppendorf pipet) are dropped onto it
- Target concentration c(rasagiline) = 5mg/10cm² or 2.25mg/4.5cm² , respectively
- Actual concentration c(rasagiline) = ca. 4.3mg/4.5cm²

| | |
|---|---|
| Durotak 87-611A (Styrene) | A liquid paddle remains on the patch |
| Durotak 87-4287 (Acrylate) | The solution permeates into the adhesive matrix, resulting in a matrix with a very soft consistency, The addition of Eudragit E hardens the matrix |
| Durotak 87-618A (PIB) | A liquid paddle remains on the patch |
| BioPSA 7-4301 | A liquid paddle remains on the patch |

### 1.3 Use of IPM in combination with acrylate/Eudragit E

- Preparation of a TDS of 4.5cm² using 20%, 40% and 60% Eudragit E in Durotak 87-4287 (RAS017, RAS018, RAS019)
- ca. 50mg of a rasagiline solution from experiment 1.1 are evenly dropped onto the TDS ( with Pasteur pipet)
- concentration c(rasagiline) = 5 mg/10 cm² or 2.25 mg/4.5 cm², respectively
- Actual concentration c(rasagiline) = ca. 1 mg/4.5 cm²

| Solvent | RAS017 Eudragit 20% | RAS018 Eudragit 40% | RAS019 Eudragit 60% |
|---|---|---|---|
| Isopropyl myristate (IPM) | Solution permeates into the matrix, very soft matrix, some residual liquid remains | Solution permeates into the matrix, very soft matrix | Solution permeates into the matrix, very soft matrix |
| Cremophor EL | Solution does not permeates but contracts | | |
| Vitamin-E-acetat | Solution does not permeates but contracts | | |
| Propylene glycol | Solution permeates, probably due to the evaporation of the propylene glycol | | |
| PEG 400 | Solution does not permeates but contracts | | |
| Glycerol, anhydrous | Solution does not permeates but contracts | | |
| paraffine, viscous | Solution does not permeates but contracts | | |
| Polysorbat 80 | Solution does not permeates but contracts | | |

### 1.4 Use of silicone oil in combination with BioPSA 7-4301

- BioPSA 7-4301: A TDS of 4,5cm² is punched out
- ca. 50mg of a rasagiline solution from experiment 1.1 are evenly dropped onto the TDS ( with Pasteur pipet)
- Target concentration c(rasagiline) = 5 mg/10 cm² or 2.25 mg/4.5 cm², respectively
- Actual concentration c(rasagiline) = ca. 1 mg/4.5 cm²
- Result: Solution permeates into the matrix, resulting in a very soft matrix

### 1.5 Further solubility experiments using IPM and "Silicone oil 1000"

- rasagiline base solubilised in IPM: Solubility > 200mg rasagiline/g IPM Used concentration: 51,1mg rasagiline in 258,4mg IPM (higher concentration compared to Example 1.3)
- Rasagiline IPM solution ca. 7mg applied on acrylate/EPO-TDS
- Target concentration c(rasagiline) = 5 mg/10 cm² or 2.25 mg/4.5 cm², respectively
- Actual concentration c(rasagiline) = ca. 1 mg/4.5 cm²

| | Eudragit 20% | Eudragit 40% | Eudragit 60% |
|---|---|---|---|
| Isopropyl myristate (IPM) | Good permeation of the solution, soft/sticky matrix | | Good permeation of the solution, firm and non-adhesive matrix |

- rasagiline base solubilised in silicone oil: solubility ca. 25-30mg/g silicone oil 1000
- Rasagline silicone oil solution 5mg/10mg/20mg/40mg applied onto a 4.5cm² TDS patch (BioPSA 7-4301)
- Target concentration c(rasagiline) = 5 mg/10 cm² or 2.25 mg/4.5 cm², respectively
- Actual concentration c(rasagiline) = ca. 0.125 - 1mg/4.5 cm²

### (Obviously to low, ca. 90mg are expected)

| Silicone oil 1000 | BioPSA 7-4301 |
|---|---|
| 5mg (0,125mg rasagiline) | Sticky, not to soft |
| 10mg (0,25mg rasagiline) | Sticky, not to soft |
| 20mg (0,5mg rasagiline) | Sticky, not to soft |
| 40mg (1mg rasagiline) | Very soft matrix |

### 1.6 Thickening of the rasagiline IPM solution using Eudragit E

- After addition of Eudragit E to the rasagiline solution (ca. 1:1 and 2:3) the mixture thickens, and becomes pasty, can not be streaked
- Comment:
   ■ the thickened solution should applied/laminated on the dried adhesive matrix
   ■ for a solid matrix a cross-linked adhesive should be used(e.g. Durotak 2516)

### 1.7 Concentrating of rasagiline in IPM

- rasagiline base solubilised in IPM: solubility ca. 1g/g IPM actual concentration: 50,2mg rasagiline in 53,2mg IPM
- rasagiline is solubilised, yielding a yellow solution
- Allow to stand over night: rasagiline is still solubilised, solution has a more intense yellow colour
- Stirring shortly , rasagiline crystallizes from the solution
- Crystals dissolve due to short heating

### 1.8 Application of rasagiline IPM solution onto acrylate-Eudragit E-TDS

### Pilot experiment:

IPM is applied on a 10cm² TDS, can be spread over the complete TDS

### Experiment 1.8a:

- 1 part rasagiline is solubilised in 3 parts of IPM resulting in a concentration of 5mg/10cm²
- The solution is stirred for 15min
- 20mg of the solution are applied onto a TDS of 10cm², dispersed with a small spatula and the exact applied quantity is tared
- The patch is allowed to stand for 30min, so that the solution can permeates;
   afterwards the patch is laminated with FL 2000
- RAS 017 TS_D001 with 5mg rasagiline, 15mg IPM n=3, the actual content can be found in the manufacturing protocol "RAS 020 TS_D001"
- RAS 018 TS_D001 with 5mg rasagiline, 15mg IPM n=3
- RAS 019 TS_D001 with 5mg rasagiline, 15mg IPM n=2
- The patches are analysed 3 days after preparation for crystals.
Result: Only sporadic crystals were observed, macroscopically the patches exhibit a wave-like structure

### Experiment 1.8b:

- 1 part rasagiline is solubilised in 9 parts of IPM resulting in a concentration of 5mg/10cm²
- The solution is stirred for 15min
- 20mg of the solution are applied onto a TDS of 10cm², dispersed with a small spatula and the exact applied quantity is tared
- The patch is allowed to stand for 60min, so that the solution can permeates; afterwards the patch is laminated with FL 2000
- RAS 017 TS_D001 with 5mg rasagiline, 45mg IPM n=1
- RAS 018 TS_D001 with 5mg rasagiline, 45mg IPM n=1
- RAS 019 TS_D001 with 5mg rasagiline, 45mg IPM n=1
- The patches are analysed 3 days after preparation for crystals.
Result: Only sporadic crystals were observed, macroscopically the patches exhibit a prominent wave-like structure

### 1. 9 Application of a rasagiline IPM solution onto acrylate-Plastoid B-TDS

Preparation of a 10cm² TDS with 20%, 40% and 60% Plastoid B in Durotak 87-4287 (RAS021, RAS022, RAS023)
- 1 part rasagiline is solubilised in 3 parts of IPM resulting in a concentration of 5mg/10cm²
- The solution is stirred for 15min
- 20mg of the solution are applied onto a TDS of 10cm², dispersed with a small spatula and the exact applied quantity is tared
- The patch is allowed to stand for 60min, so that the solution can permeates; afterwards the patch is laminated with FL 2000
- RAS 021 TS_D001 with 5mg rasagiline, 15mg IPM n=3
- Result: The matrix is solid and adhesive
- RAS 022 TS_D001 with 5mg rasagiline, 15mg IPM n=3
- Result: The matrix is solid but less adhesive than RAS 021
- RAS 023 TS_D001 with 5mg rasagiline, 15mg IPM n=3
- Result: The matrix is solid but less adhesive than RAS 021
- The patches are analysed 4 days after preparation for crystals.
- Result: Only sporadic crystals were observed, macroscopically the patches exhibit a slight wave-like structure
- From every batch 6 TDS patches with an area of 1.5cm² are punched out and analysed for in vitro skin permeation

Result: Patch from exp. 1.9 shows superior skin permeation compared to patch from exp. 1.8. Analysis of the patches RAS021-023 in skin permeability revealed a high cumulative permeation rate for all three patches (see **Figure 4**).

### 1.10 Sublimation of rasagiline base at 60-70°C

### Rasagiline is melted in the beaker glass at 60-70°C and covered with an ice-cold watch glass

### Result: The rasagiline re-sublimates on the cooled watch glass.

### Conclusion:

A processing of the rasagiline in volatile/low boiling solvents is not possible. Even a slight increase in temperature results in a sublimation/evaporation of the substance.

The most promising strategy includes the preparation of a placebo patch with subsequent application of a rasagiline solution.

In order to realize this strategy, an additional absorbent polymer has to be added to the PSA. For this purpose Plastoid B/Eudragit polymers and the like are preferably suited.

Suitable solvents are IPM and silicone oil. However, a relatively high concentration of rasagiline in the solvent is required, preferably a conc. ≥ 50%, in order to restrict the smoothening of the patch and to allow a proper soakening. IPM proved out to be a successful solvent.

### EXAMPLE 2 Testing of further solvents

### Objective

It should be analysed if Polidocanol is as good as IPM for application of rasagiline.

### Description

The strategy of applying a rasagiline solution onto a placebo patch (particularly by dropping onto the patch) yielded good results. The solvent IPM has the disadvantage of being a penetration enhancer. Alternative solvents should be identified that lack a penetration enhancing effect.

### Methods

### 2.1 Application of ca. 20mg Polidocanol onto the placebo patches RAS021 TS_D001, RAS022TS_D001, or RAS023TS_D001.

**Result:** Polidocanol permeates the patch.

### 2.2 Solubility of rasagiline in Polidocanol

Mixing of 48.5mg rasagiline and 147.0mg polidocanol
**Result:** rasagiline is completely soluble.

### 3.3 Preparation of placebo laminates using Plastoid B

Siehe manufacturing protocol for RAS032TS_D001, RAS033TS_D001 or RAS034TS_D001
- From the prepared placebo laminates TDS patches with an area of 10cm² were punched out
- ca. 20mg of a rasagiline polidocanol (1:3) solution are applied, target concentration: c(rasagiline)=5mg/TDS
- the patch is allowed to stand for 60min, during this time the solution permeates the patch
- thereafter the Patch is laminated with a back up layer (FL2000 Liner)
- after the residence time, TDS with an area of 1.5cm² are punched out for in vitro analysis of skin permeation

### Aspect of the patches:

- RAS032TS_D001 (20% Plastoid B): adhesive, slightly slumped, slightly fatty TDS without wave-like structure
- RAS033TS_D001 (40% Plastoid B): sparsely adhesive, slightly slumped, slightly fatty TDS without wave-like structure
- RAS034TS_D001 (60% Plastoid B): non adhesive to sparsely adhesive, slightly slumped, slightly fatty TDS without wave-like structure

### Conclusion

Based on the results polidocanol is a suitable solvent for rasagiline. Furthermore the solvent completely permeates the patch, which constitutes an ideal requirement fort he further development. It is known that polidocanol is hardly released from an acrylate matrix. Therefore it can be expected that no or only a limited co-diffusion of rasagiline and polidocanol will take place.

The prototypes RAS033TS_D001 and RAS034TS_D001 were further analysed in a skin permeation experiment and revealed an excellent cumulative skin permeation rate that was even higher as the permeation rate observed for the Plastoid-containing patches (see Figure 5). Due to the fact that polidocanol exhibits pharmacological effects, additional experiments were undertaken to identify structural similar solvents of high molecular weight.

### EXAMPLE 3: Preparation and testing of a two-layer prototype

### Background

In pre-tests the Plastoid B was initially mixed with the adhesive matrix. This resulted in patches with a very little adhesive force. In order to overcome this problem the strategy of a multiple layer prototype was tested build up from an adhesive layer and a plastoid layer.

### Methods

The plastoid layer is created using a 2% Miglyol solution. Therefore 0.1 g Plastoid B (WE8656) were solubilised in Miglyol 812 (WE8884) ad 5,0 g.

As an adhesive layer DuroTak 4287 (WE7984, solid content ∼38%) is used. The adhesive is streaked onto FL2000 in a way that the dry area-weight equates ca. 60 g/m². The layers are dried at room temperature for 10 min, followed by 30 min at 70°C. A web was streaked and the resulting laminate bisected.

For the application of the Plastoid two variants were tested:
(1) The plastoid-layer is directly coated onto the dried adhesive streak. The coating weight accounts for 40g/m². The laminate was dried for 10 to 60 min at RT. The resulting laminate was coated with Hostaphan RN15 (WE6776).
(2) The plastoid layer is streaked onto Hostaphan RN15. The coating weight accounts for 40g/m². The laminate was dried for 10 min to become touch dry and further coated with an adhesive layer.

### Processing and laminate aspect were evaluated.

### Results

**Experiment 3.1:** Plastoid does not solubilize as 2% solution in miglyol. The plastoid remain as unsolubilised crystals. In further experiments (see exp.; 3.2 and 3.3) the solution as warmed with caution while stirring to a temperature of ca. 50°C. The plastoid dissolves and remains solubilised. The resulting solution exhibits a very low viscosity.

**Experiment 3.2:** Herein Eudragit E was used for a muli-layer TDS. Eudragit E and Miglyol were weighted in a ratio of 1:1 in an aluminium dish and mixed with a Pasteur pipet. The mixture which was very dry in the beginning liquefied during the time to result in a viscous clear mass (viscosity similar to viscous honey).

**Experiment 3.3:** Plastoid B and Miglyol were weighted in a ratio of 1:1 in an aluminium dish and mixed which resulted in a crystalline mass. This was heated in a heating oven at 50°C yielding a clear "clump". After addition of Miglyol to obtain a ration of 1:2 and further heating at 50°C, a viscous clear mass similar to exp. 3.2. was obtained (viscosity similar to viscous honey).

**Experiment 3.4:** A 12% Plastoid B solution as prepared. This solution was stirred at ca. 50°C until the Plastoid was completely dissolved. After 3 hours the solution was clear, however, undissolved plastoid particles could be seen at the border of the glass. The viscosity of the resulting solution is too low to be streaked (even after cooling). After two days of holding time the solution becomes more viscous.

**Experiment 3.5:** A 25% Plastoid B solution as prepared. This solution was stirred at ca. 50 to 60°C until the Plastoid was completely dissolved. After 2.5 hours the solution was clear, no plastoid particles could be observed. Accordingly neutral oil was added. The resulting 20% solution still exhibits a high viscosity. It was found that a solution of 16.67 % possesses an acceptable viscosity. This solution was further processed in experiment 3.6.

**Experiment 3.6:** A placebo laminate was prepared by streaking Durotak 4287 with a dry area weight of 60g/m².

Web No 1. R.III 200µm dry area weight of 69.177g/m²
(1) Plastoid solution was coated onto a dried adhesive layer with a thickness of 110µm, dried at RT for 60min. Total area weight = 103.115g/m² (rel. std. dev. 9.5%); Aspect: very soft matrix - in experiment 3.7 it should be tried to solidify the matrix by increasing the plastoid concentration
(2) Not possible, since the plastoid solution is smearing during the coating process

### Experiment 3.7:

The batch RAS035TS_D001 was prepared. The plastoid concentration in the final laminate should equates 20%.
   - Area weight adhesive matrix = 70g/m²
   - Total area weight: 120g/m² (rel. std. dev. 13.8%)
   - Theoretical area weight neutral oil-plastoid: 50g/m²
   - Aspect: clear an homogenous laminate, soft matrix

### Experiment 3.8: rasagiline solubility in a plastoid neutral oil solution

- 50mg rasagiline WE 8596 in 300mg Plastoid-solution from exp. 3.5
- Rasagiline is dissolved after 10min, resulting in a clear, slightly yellow solution

### Experiment 3.9: Placebo-TDS with plastoid-neutral oil solution

The batch RAS036TS_D001 was prepared by using three different concentrations of plastoid B (12.5%, 22.5% or 32.5%). The coating of the neutral oil-plastoid solution is performed according to the manufacturer's protocol:
The FL2000 layer is aspirated with the non-siliconized side onto the coating plate. The adhesive laminate pieces are attached with an adhesive tape on the liner, so that centrally and at the edges an open area is produced allowing a free guidance of the scraper. The scraper adjustment is calculated based on the foil thickness, area weight of the adhesive matrix, plus the targeted area weight of the plastoid neutral oil solution Targeted area weight of the adhesive matrix 80g/m² Targeted total area weight (Placebo) 95g/m²

### 12,5% Plastoid

- Area weight of the adhesive matrix: 78.577g/m²
- Total area weight: 94.128g/m² (rel. std dev 1.14%)
- Theoretical area weight of the plastoid neutral oil solution: 15.55g/m²
- Aspect: clear homogenous laminate, detach from the skin like chewing gum

### 22,5% Plastoid

- Area weight of the adhesive matrix: 76.143 g/m²
- Total area weight: 89.092 g/m² (rel. std. dev. 2.02%)
- Theoretical area weight of the plastoid neutral oil solution: 12.95g/m²
- Aspect: clear homogenous laminate

### 32,5% Plastoid

- Area weight of the adhesive matrix: 80.334 g/m²
- Total area weight: 97.670 g/m² (rel. std dev. 5.88%)
- Theoretical area weight of the plastoid neutral oil solution: 17.34 g/m²
- Aspect: clear homogenous laminate

### Experiment 3.10: Rasagiline TDS with a Plastoid-neutral oil solution

Since the laminates produced in exp. 3.9 possess good adhesive properties they were produced as API containing patches of the following batches: RAS037TS_D001 (32,5% Plastoid); RAS038TS_D001 (22,5% Plastoid)

### Preparation protocol see example 6

Targeted area weight of the adhesive matrix 80 g/m²
Targeted total area weight (Placebo) 100 g/m²

### RAS037TS_D001

- Area weight of the adhesive matrix: 84,559g /m²
- Total area weight: 101.243g/m² (rel. Std dev 12.40%)
- Theoretical area weight of the plastoid neutral oil solution: 16.684 g/m²
- Aspect: clear homogenous laminate

### RAS038TS_D001

- Area weight of the adhesive matrix: 82,452 g/m²
- Total area weight: 101.463 g/m² (rel. Std dev 4.08 %)
- Theoretical area weight of the plastoid neutral oil solution: 19.011 g/m²
- Aspect: clear homogenous laminate

From each batch 6 patches were analysed in an in vitro skin permeation assay. The batch RAS022TS_D001 was used as reference.

### Conclusion

The rasagiline TDS resulted at both plastoid concentrations in a good aspect.

### EXAMPLE 4: Testing of macrogol ether and -ester as solvent

### Background

Previous experiments showed that polidocanol is a very good solvent for rasagiline. However, the polidocanol possesses a pharmaceutical effect (antipruritic) which could pose regulatory problems. Therefore polidocanol analogues with longer chains and lacking pharmacological effects were analysed. Furthermore also macrogol esters were tested. Since these substances are solid compounds it was tried to melt them for further processing.

### Method

It was tried to liquefy the compounds Brij S20, Brij CS20, Crodet S40 und Cithrol 10 MS at a temperature of 40°C.

Ca. 0.5 g were weighted in a beaker glass and heated on a heating plate up to 40°C while the melting characteristics of the compounds were observed.

### Results

Ca. 0.5 g were weighted in a beaker glass and heated on a heating plate while stirring. The heating plate had a temperature of ca. 65-70°C, resulting in a melting temperature of ca. 40-50°C within the glass.

| Solid compound | Appearance at ca. 40°C | Comment |
|---|---|---|
| Brij S20 (Pellet's) | Melts quickly: ca. 1 min | |
| Brij CS20 (Pellet's) | Melts within ca. 3 min | |
| Crodet S40 (Pellet's) | Melts within 5 min | Platte was not so hot anymore: ca. 55°C |
| Cithrol 10 MS (compound is solid, small particles were scrapped from the block) | Melts quickly: ca. 1 min | |

| | | |
|---|---|---|
| Within 1-2 minutes all compounds become solid again. | | |

### Conclusion

All solid compounds can be melted at a low temperature therefore permitting a processing. However, the experiment shows also that the molten compounds will quickly become solid again when the temperature is lowered.

### EXAMPLE 5: Placebo and rasagiline TDS with macrogol derivates

### Goal

The solubility of the rasagiline in the macrogol derivatives should be analysed and acrylate patches should be prepared using a placebo or an API-containing solution.

### Methods

The above listed substances were melted at 40°C. From each molten substance 20 mg were dropped onto a placebo acrylate patch and dispersed. As placebo patch rectangular 10 cm² die-cut sheets from the laminate RAS022TS_D001 were used. The handling and the soaking characteristics were documented. Macrogol derivates with good processability were analysed as follows:
210 mg of the macrogol derivate was molten and 70 mg rasagiline were added. The salvation characteristics of the rasagiline was observed and documented. The solvation process has to be performed under stirring.

API containing patches were produced using the macrogol derivates with good solubility of rasagiline. The handling and the soaking characteristics were documented.

Die resulting patches were analysed for skin permeation, for content/purity or presence of crystals.

### Results

| Macrogol derivates | comment / description |
|---|---|
| Cithrol 10 MS | The molten macrogol derivative solidifies during the application on the TDS. It can't be dissolved evenly.. |
| Brij S20 | Forceps were warmed for application. The application is more practical, also here the macrogol solidifies quickly. |
| Both samples were cautiously warmed on a heating plate whereby the magrogol melts again and the derivative can be dispersed. Both TDS samples were kept for ca. 45 minutes on the heating plate at this temperature. The macrogol derivatives contract and generate drops but do not permeate the adhesive matrix. After cooling of the patches the macrogol derivates solidify again creating a wax-like sheet on the TDS. | |
| Brij CS20 | Forceps were warmed for application. The application is more practical, also here the macrogol solidifies quickly. |
| Crodet S40 | Forceps were warmed for application. The application is more practical, also here the macrogol solidifies quickly. |
| Both samples were cautiously warmed on a heating plate whereby the magrogol melts again and the derivative can be dispersed. Both TDS samples were kept for ca. 30 minutes on the heating plate at this temperature. The macrogol derivatives contract and generate drops but do not permeate the adhesive matrix. After cooling of the patches the macrogol derivates solidify again creating a wax-like sheet on the TDS. | |

For the following experiments the pipets and the placebo TDS were pre-warmed in a drying oven. The TDS batch RAS022TS (round, area of 10 cm²) was used.

### Experiment 5.1:

70mg rasagiline was weighted into a small screw cap glass. Cithrol 10MS was melted in a drying oven at 50°C and 210 mg molten Cithrol was added with the pre-warmed pipet to the rasagiline.

### Observation: During the addition the rasagiline melts. After cooling the mixture solidifies quickly.

The mixture of rasagiline and Cithrol is melted for 2 minutes in the drying oven and homogenized subsequently for ca. 1 minute. 20 mg of this mixture is applied on to a pre-warmed placebo and dispersed using a Pasteur pipet. The TDS is covered and cooled. The rasagiline-macrogol derivate mixture solidifies unevenly and criss-crosses on the TDS.

### Experiment 5.2:

70mg rasagiline was weighted into a small screw cap glass. Brij S20 was melted in a drying oven at 50°C and 210 mg molten Brij S20 was added with the pre-warmed pipet to the rasagiline.

Result: The added Brij S20 solidifies more quickly, resulting in a "wax-like drop". The mixture is melted in a drying oven, whereby the rasagiline is molten within 2 minutes and the Brij within further 7 minutes. The molten mixture is homogenized subsequently for ca. 1 minute. 20 mg of this mixture is applied on to a pre-warmed placebo and dispersed using a Pasteur pipet whereby the mixture solidifies very quickly. The mixture is melted again in the drying oven and dispersed again. The TDS is covered and cooled. The rasagiline-macrogol derivate mixture solidifies quickly but disperses more evenly and was coated with a adhesive silicone matrix.

### Experiment 5.3:

70mg rasagiline was weighted into a small screw cap glass. CrodetS40 was melted in a drying oven at 55°C and 210 mg molten CrodetS40 was added with the pre-warmed pipet to the rasagiline.

### Result:

The added CrodetS40 solidifies very quickly, resulting in a "wax-like drop". The mixture is melted in a drying oven, whereby the rasagiline is molten within 2 minutes and the Crodet within further 7 minutes. The molten mixture is homogenized subsequently for ca. 1 minute.

20 mg of this mixture is applied on to a pre-warmed placebo and dispersed using a Pasteur pipet whereby the mixture solidifies very quickly. As a result it was not possible to disperse the mixture evenly on the TDS. The mixture is melted again in the drying oven and dispersed again. The TDS is covered and cooled. The rasagiline-macrogol derivate mixture solidifies quickly but disperses more evenly and was coated with an adhesive silicone matrix.

As a reference fort he skin permeation experiments, a "fresh" TDS using IPM as solvent was prepared. Therefore 70 mg rasagiline were solubilised in IPM. 20 mg of this solution were applied on a 10 cm² acrylate patch and dispersed evenly. (In analogy to example 1).

### Conclusion

It is possible to produce TDS patches by slight warming treatment. BrijS20 and Crodet are particularly suited as solvents for rasagiline. Since the macrogol derivates form a wax-like sheet, they are coated with a thin silicone layer (ca. 20G/m²) as "skin streak". The samples with the following batch numbers wee tested for skin permeation:
- RAS022TS_D001_BrijS20 (content rasagiline: 0,63 mg/1,5 cm²)
- RAS022TS_D001_Crodet (content rasagiline: 0,68 mg/1,5 cm²)

As reference a freshly prepared patch of the same batch without silicone layer with IPM as solvent was used:
- RAS022TS_D001_IPM (content rasagiline: 0,84 mg/1,5 cm²)

Analysis of the patches RAS022TD containing Brij or Crodet regarding skin permeability revealed a high cumulative permeation rate for both patches which is similar to the Plastoid containing reference patch (see Figure 4).

### EXAMPLE 6: Preparation of a rasagiline multilayer TDS

### Goal

Based on a solution of rasagiline in neutral oil and Plastoid B a multi-layer TDS should be prepared.

### Methods

The following substances were mixed to generate Mixture A:
- 0,35 g rasagiline base
- 0.175 g Plastoid B
- 0.875 g Neutral oil

The following substances were mixed to generate **Mixture B:**
- 9,035 g Durotek 87-4287
- 2.1 g Plastoid B
- 7.465 g Ethylacetat

The preparation of the patch includes the following steps: The adhesive laminate pieces are attached with an adhesive tape on the liner, so that centrally and at the edges an open area is produced allowing a free guidance of the scraper.
- The liner is aspirated onto the coating plate (mixture B)
- The adhesive laminate pieces are attached with an adhesive tape on the liner, so that centrally and at the edges an open area is produced allowing a free guidance of the scraper.
- Scraper adjustment: 210 µm
- Rasagiline solution (Mixture A) is applied on the adhesive layer
- Solution permeates for 60 minutes in the dark
- Laminate is coated with a back up layer (Trespaphan)
- Samples are punched out for skin permeation experiments

Thus, the patches according to the present invention having an area of about 1 to 50 cm² were able to transdermally provide a therapeutic dose needed for up to 7 days treatment, and the penetration rate of rasagiline could be controlled by using multilayer patches. Since the raw materials for preparing the patches are widely and readily available, and rasagiline could be transferred through the whole surface of the substrate for transdermal absorption, the patches of the present invention could be prepared simply with widely available raw materials, have smooth release profiles and a prolonged period for controlled release, and may be more effectively absorbed through skin in comparison with the system as described in WO 2007/101400.

## Claims

1. A method for preparing a transdermal patch comprising a substrate layer, the method comprising the steps of:
a) contacting an active pharmaceutical ingredient with a retaining means to provide a composition; and
b) applying the composition obtained in step (a) to a carrier material to form a substrate layer of the transdermal patch;
wherein the retaining means remains within the final transdermal patch.

2. The method according to claim 1, wherein the active pharmaceutical ingredient has a chemical structure according to one of the following formulas: 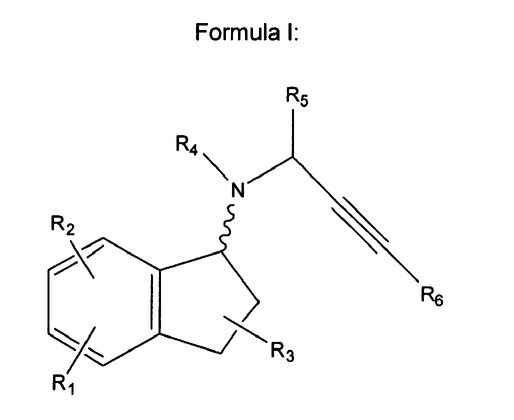 wherein
R₁ is hydrogen, halogen, alkyl, alkoxy, acyl, acyloxy, aryl, aralkyl, hydroxy, carboxy, amine, alkylamine, dialkylamine, nitro, or -OC(O)NR₁₂R₁₃, and may be substituted by one or more substituents selected from alkyl, halogen, hydroxy, carboxy, amine, alkylamine, dialkylamine;
R₂, R₃, R₄, R₅, R₆, R₁₂ and R₁₃ independent from each other are hydrogen, halogen or alkyl;
and wherein one or more, preferably one or two, of the carbon atoms in the formula including the substituents may be replaced by a heteroatom such as nitrogen, oxygen or sulfur; and
Formula II: 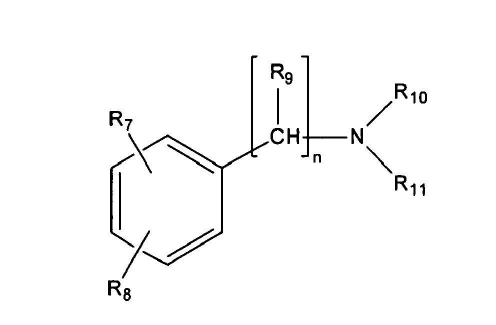 wherein
R₇ is hydrogen, halogen, alkyl, alkoxy, acyl, acyloxy, aryl, aralkyl, hydroxy, carboxy, amine, alkylamine, dialkylamine, nitro, or -OC(O)NR₁₄R₁₅, and may be substituted by one or more substituents selected from alkyl, halogen, hydroxy, carboxy, amine, alkylamine, dialkylamine;
R₈, R₉, R₁₀, R₁₄ and R₁₅ independent from each other are hydrogen, halogen or alkyl, wherein if more than one R₉ is present, these R₉ groups may also be different from each other;
R₁₁ is hydrogen, halogen or alkyl optionally substituted by halogen or hydroxy;
n is an integer from 1 to 4, preferably 1 or 2;
and wherein one or more, preferably one or two, of the carbon atoms in the formula including the substituents may be replaced by a heteroatom such as nitrogen, oxygen or sulfur.

3. The method according to claim 1 or 2, wherein the active pharmaceutical ingredient is a volatile substance and preferably is rasagiline, selegiline, rivastigmine or ladostigil, or a derivative thereof, preferably in the form of the free base.

4. The method according to any one of claims 1 to 3, wherein the retaining means is a liquid, preferably a pharmaceutically acceptable low volatile solvent, more preferably selected from the group consisting of synthetic or natural oils, isopropyl myristate, polyethoxylated fatty acids and polyethoxylated fatty alcohols such as polidocanol, Brij-, Crodet-, Myrj,-,Atlas-types, and mixtures thereof, and the active pharmaceutical ingredient is contacted with the retaining means in step (a) to form a solution, suspension or emulsion.

5. The method according to claim 4, wherein a soaking additive is further added to the either the carrier material or the drug solution.

6. The method according to any one of claims 1 to 5, wherein the retaining means is a plasticizer and the carrier material is a mixture of an adhesive polymer and a non-adhesive polymer.

7. The method according to any one of claims 1 to 3, wherein the retaining means is solid, preferably selected from the group consisting of higher molecular weight polyethoxylated fatty acids and polyethoxylated fatty alcohols, PVP, PEO, PVA, PVPVA, cellulose and derivatives, starch and derivatives and their blends.

8. The method according to claim 7, wherein the active pharmaceutical ingredient is contacted with the retaining means in step (a) in a molten state, mixed, solidified and ground to form a solid mixture.

9. The method according to claim 7, wherein the active pharmaceutical ingredient is attached to the surface of the retaining means to form a coated or impregnated material.

10. The method according to any one of claims 1 to 9, wherein the carrier material comprises at least one type of the following polymer materials: polyacrylates and derivatives thereof, silicone polymers and derivatives thereof, polyisobutylene and derivatives thereof, ethylene-vinyl acetate copolymers and derivatives thereof, styrene-block-co-polymers and derivatives thereof, POX and derivatives thereof, polyurethanes and derivatives thereof, polyolefines and derivatives thereof, polyesters and derivatives thereof, and polyacrylic acids and derivatives thereof.

11. The method according to any one of claims 1 to 10, wherein the carrier material is an adhesive, preferably an adhesive acrylate, polyurethane, polyisobutylene or styrene-block-co-polymer.

12. The method according to any one of claims 1 to 11, wherein the carrier material contains a soaking additive, preferably Plastoid B or Eudragit.

13. The method according to any one of claims 1 to 12, wherein the carrier material contains a non-adhesive, preferably a non-adhesive acrylate or a fleece material, and an adhesive layer is further introduced to form the transdermal patch.

14. The method according to any one of claims 1 to 13, wherein the transdermal patch further comprises a backing layer, a protective layer and/or a release controlling layer such as a membrane having a defined pore size.

15. The method according to any one of claims 1 to 14, wherein the method comprises the following steps:
a) adsorbing the active pharmaceutical ingredient onto the solid retaining means to form a composition, preferably by melting the active pharmaceutical ingredient in presence of the retaining means and then solidifying;
b) grinding the composition;
c) mixing the ground composition with molten carrier material or with a solution comprising the carrier material;
d) applying the dispersion or solution onto a backing layer to form a film;
and
e) cooling or drying the film to form a substrate layer.

16. The method according to any one of claims 1 to 14, wherein the method comprises the following steps:
a) forming an adhesive matrix layer comprising the carrier material;
b) mixing the active pharmaceutical ingredient with the liquid retaining means to form a solution or suspension;
c) applying the solution or suspension to the matrix layer to form a substrate layer, preferably by soaking, printing or casting.

17. The method according to any one of claims 1 to 14, wherein the method comprises the following steps:
a) forming an adhesive matrix layer comprising the carrier material;
b) mixing the active pharmaceutical ingredient with the liquid retaining means and a non-adhesive carrier material to form a viscous solution or suspension;
c) applying the solution or suspension to the matrix layer to form a substrate layer, preferably by spreading; and
d) optionally applying another adhesive layer.

18. The method according to any one of claims 1 to 14, wherein the method comprises the following steps:
a) forming an adhesive matrix layer comprising the carrier material;
b) attaching the solid retaining means, preferably a fleece material, to the adhesive matrix layer;
c) applying the active pharmaceutical ingredient to the retaining means, for example by impregnating, either before or after the retaining means is attached to the adhesive matrix layer; and
d) applying another adhesive layer to the other site of the retaining means.

19. The method according to any one of claims 1 to 14, wherein the method comprises the following steps:
a) forming an adhesive matrix layer comprising the carrier material;
b) adsorbing the active pharmaceutical ingredient onto the solid retaining means to form a composition, preferably by melting the active pharmaceutical ingredient in presence of the retaining means and then solidifying;
c) grinding the composition;
d) applying the ground composition to the adhesive matrix layer to form a substrate layer; and
e) optionally applying another adhesive layer.

20. A method for preparing a transdermal patch comprising a substrate layer, the method comprising the steps of:
a) melting a carrier material;
b) quickly dispersing an active pharmaceutical ingredient into the molten carrier material;
c) forming a substrate layer with the mixture obtained in step (a); and
d) quickly cooling the substrate layer to solidify it.

21. The method according to claim 20, wherein the carrier material is an adhesive, the substrate layer is formed on a backing layer and/or the substrate layer is cooled by forming it on a cooled surface.

22. A transdermal patch comprising a backing layer and a substrate layer comprising a volatile active pharmaceutical ingredient, a carrier material and a retaining agent.

23. The transdermal patch according to claim 22, which is obtainable by a method according to any one of claims 1 to 21.

24. The transdermal patch according to claim 22 or 23, wherein the active pharmaceutical ingredient has a chemical structure according to one of the following formulas: 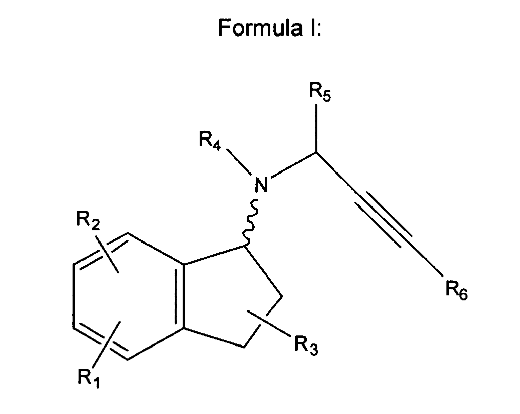 wherein
R₁ is hydrogen, halogen, alkyl, alkoxy, acyl, acyloxy, aryl, aralkyl, hydroxy, carboxy, amine, alkylamine, dialkylamine, nitro, or -OC(O)NR₁₂R₁₃, and may be substituted by one or more substituents selected from alkyl, halogen, hydroxy, carboxy, amine, alkylamine, dialkylamine;
R₂, R₃, R₄, R₅, R₆, R₁₂ and R₁₃ independent from each other are hydrogen, halogen or alkyl;
and wherein one or more, preferably one or two, of the carbon atoms in the formula including the substituents may be replaced by a heteroatom such as nitrogen, oxygen or sulfur; and 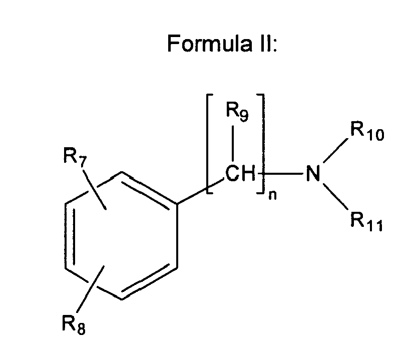 wherein
R₇ is hydrogen, halogen, alkyl, alkoxy, acyl, acyloxy, aryl, aralkyl, hydroxy, carboxy, amine, alkylamine, dialkylamine, nitro, or -OC(O)NR₁₄R₁₅, and may be substituted by one or more substituents selected from alkyl, halogen, hydroxy, carboxy, amine, alkylamine, dialkylamine;
R₈, R₉, R₁₀, R₁₄ and R₁₅ independent from each other are hydrogen, halogen or alkyl, wherein if more than one R₉ is present, these R₉ groups may also be different from each other;
R₁₁ is hydrogen, halogen or alkyl optionally substituted by halogen or hydroxy;
n is an integer from 1 to 4, preferably 1 or 2;
and wherein one or more, preferably one or two, of the carbon atoms in the formula including the substituents may be replaced by a heteroatom such as nitrogen, oxygen or sulfur.

25. The transdermal patch according to any one of claims 22 to 24, wherein the active pharmaceutical ingredient is a volatile substance and preferably is rasagiline, selegiline, rivastigmine or ladostigil, or a derivative thereof, preferably in the form of the free base.

26. The transdermal patch according to any one of claims 22 to 25, wherein the retaining means is a liquid, preferably a pharmaceutically acceptable low volatile solvent, more preferably selected from the group consisting of natural or synthetic oils, fatty acid esters, such as isopropyl myristate, polyethoxylated fatty acids and polyethoxylated fatty alcohols such as polidocanol, and mixtures thereof.

27. The transdermal patch according to any one of claims 22 to 26, wherein the retaining means is a plasticizer and the carrier material is a mixture of an adhesive polymer and a non-adhesive polymer.

28. The transdermal patch according to any one of claims 22 to 25, wherein the retaining means is a solid, preferably a pharmaceutically acceptable solid, more preferably selected from the group consisting of higher molecular weight polyethoxylated fatty acids and polyethoxylated fatty alcohols, PVP, PVA, PVPVA, PEO, cellulose and derivatives, starch and derivatives or their blends.

29. The transdermal patch according to any one of claims 22 to 28, wherein the transdermal patch contains at least 0.1 % w/w of the retaining means.

30. The transdermal patch according to any one of claims 22 to 29, wherein the carrier material comprises at least one type of the following polymer materials:
polyacrylates and derivatives thereof, silicone polymers and derivatives thereof, polyisobutylene and derivatives thereof, ethylene-vinyl acetate copolymers and derivatives thereof, Styrene-block-co-polymers and derivatives thereof, POX and derivatives thereof, polyurethanes and derivatives thereof, polyolefines and derivatives thereof, polyesters and derivatives thereof. and polyacrylic acids and derivatives thereof.

31. The transdermal patch according to any one of claims 22 to 30, wherein the carrier material is an adhesive, preferably an adhesive acrylate, polyurethane, polyisobutylene or styrene-block-co-polymer.

32. The transdermal patch according to any one of claims 22 to 31, wherein the carrier material contains a soaking additive, preferably Plastoid B or Eudragit.

33. The transdermal patch according to any one of claims 22 to 32, wherein the carrier material contains a non-adhesive, preferably a non-adhesive acrylate or a fleece material, and the transdermal patch further comprises an adhesive layer.

34. The transdermal patch according to any one of claims 22 to 33, further comprising a backing layer, a protective layer and/or a release controlling layer such as a membrane having a defined pore size.

35. The transdermal patch according to any one of claims 22 to 34, wherein the substrate layer further comprises a non-adhesive polymer, preferably a non-adhesive acrylate, and/or a soaking additive.

36. The transdermal patch according to any one of claims 22 or 35 for use in medicine.

37. The transdermal patch according to claim 36, wherein the active pharmaceutical ingredient is rasagiline or a derivative thereof, preferably in the form of the free base, for treatment or prophylaxis of a nervous system disease, preferably a nervous system disease selected from the group consisting of Parkinson's disease, Alzheimer's disease, depression, hyperactive child syndrome, restless leg syndrome, multiple sclerosis and abstinence syndrome.
